Europäisches Patentamt

European Patent Office

Office européen des brevets

(11) Publication number: **0 208 392**
**A1**

(12) **EUROPEAN PATENT APPLICATION**

(21) Application number: 86302447.7

(22) Date of filing: 02.04.86

(51) Int. Cl.⁴: **A 61 N 5/06**
**F 21 V 29/00**

(30) Priority: 09.04.85 GB 8509104

(43) Date of publication of application:
14.01.87 Bulletin 87/3

(84) Designated Contracting States:
AT BE CH DE FR GB IT LI LU NL SE

(71) Applicant: O'Brien, Christopher David Stanley
6 Sandringham Road Arundel Park
Chichester West Sussex(GB)

(72) Inventor: O'Brien, Christopher David Stanley
6 Sandringham Road Arundel Park
Chichester West Sussex(GB)

(74) Representative: Thiemann, Peter Albert William et al,
LLOYD WISE, TREGEAR & CO. Norman House 105-109
Strand
London WC2R 0AE(GB)

(54) UV-suntanning equipment.

(57) Suntanning equipment is described comprising a first and second portions (1,7) each with ultra-violet tubes (6,9) and defining between them a space to receive a user. Air for cooling the tubes (6,9) is supplied by blower means (14) to a heat exchanger (15) where the air is cooled by heat-exchange with coolant recycled from a remote location. The air is then used to cool the tubes and the temperature of the air is sensed and the supply of coolant to the heat-exchanger is controlled in dependence upon the sensed temperature. By this control and by dumping the heat generated by the equipment at a location remote from the equipment, it is possible to control the operating temperature of the equipment to a desired value within ± 0.5°C., with consequent and unforeseen therapeutic and commercial advantages.

FIG. 2.

- 1 -

Title: <u>IMPROVEMENTS IN OR RELATING TO</u>

<u>SUNTANNING EQUIPMENT</u>

The present invention is concerned with suntanning equipment of the kind comprising a first portion and a second portion so arranged as to define between them a space for the reception of a user, each portion comprising a plurality of ultra-violet tubes arranged to subject a user when in said space to ultra-violet A and B radiation, and means for blowing air over said tubes for the purpose of cooling them. Such suntanning equipment will be hereinafter referred to as "suntanning equipment of the kind set forth".

This invention relates to suntanning equipment of the kind set forth, preferably in the form of a so-called sun bed, which may be used for therapeutic radiation and for the purpose of tanning the skin of a user supported on the sun bed. The invention also relates to solaria incorporating the suntanning equipment.

Sun beds generally comprise a base portion which in general nowadays comprises a sheet of acrylic glass on which the user is to lie and a plurality of ultra-violet lamps in the form of tubes adapted to emit ultra-violet rays mounted in channels below the sheet of acrylic glass. A canopy portion, similar to the base portion is mounted above the base portion in such a way that a user lying between the two portions may be subjected to ultra-violet rays.

In the operation of such a sun bed, the tubes and the sun bed heat up and eventually the user will start to perspire. The salts excreted during perspiration interfere with the tanning process and this may lead to uneven tanning. Furthermore, discomfort may be experienced by the user who may experience prickly heat or burning.

Radiation with ultra-violet rays is a recognised therapeutic treatment for psoriasis, acne and other skin conditions and perspiration, prickly heat and burning are to be avoided if treatment with ultra-violet radiation is to be of any value, since otherwise treatment can only be effected for very short periods.

It has therefore been proposed to cool the sun bed by blowing air in contact with the tubes and there have been many proposals for doing this. Thus GB Patent Nos. 1 540 488 and 1 540 489 describe devices for the ultra-violet radiation of a person which may comprise cooling blowers. GB Patent No. 1 567 979 describes radiation apparatus for cosmetic and photobiological purposes with ultra-violet ray sources and means for suppressing the short wave rays below 320 nm and for suppressing the longer wave rays above 800 nm. The specification describes that air may be forced past the ultra-violet ray lamps and that suitably arranged cooling may be provided without, however, giving any details of how such cooling may be provided. GB Patent Specification 2 077 897A describes a sun bed with a fan for blowing cooling air over a bank of starter coils for a bank of ultra-violet tubes.

While the need for cooling the ultra-violet tubes has long been recognised, so far as I have been able to ascertain, this cooling has been on an ad hoc and haphazard basis. I have now discovered that by carefully controlling the cooling and effecting the cooling in a particular manner a number of unexpected therapeutic, technical and commercial advantages arise in a manner which could not have been foreseen.

According to one aspect of the present invention there is provided a method of operating suntanning equipment of the kind set forth, comprising the steps of

1) blowing air through heat-exchange means to cool the air,

2) passing the cooled air in contact with the ultra-violet tubes to cool the latter,

3) passing cooled coolant to said heat-exchange means for cooling said air by heat-exchange therewith,

4)      passing coolant warmed by such heat-exchange to
        cooling means,

5)      providing said cooling means at a location
        remote from the atmosphere ambient to the
        suntanning equipment,

6)      sensing the temperature of the air cooling the
        ultra-violet tubes, and

7)      controlling the supply of cooled coolant to said
        heat-exchange means in dependence upon the
        sensed temperature.

According to another aspect of the present
invention there is provided suntanning equipment,
comprising a first portion, a second portion, the
portions being arranged so as to define between them a
space for the reception of a user, a first plurality of
ultra-violet tubes mounted in said first portion, a
second plurality of ultra-violet tubes mounted in said
second portion means for operating said ultra-violet
tubes, whereby a user can be subjected to ultra-violet
radiation when in said space, means for blowing air
over said tubes for the purpose of cooling them,
heat-exchange means to cool the air suplied by said
air-blowing means, cooling means for providing a supply
of cooled coolant, said cooling means being located
remote from the atmosphere ambient to said equipment,
first conduit means for feeding cooled coolant to said heat-exchange means, second conduit means for recycling coolant from said heat-exchange means back to said cooling means, control valve means in said first conduit means for regulating the supply of cooled coolant to said heat-exchange means, and .temperature sensing means to sense the temperature of the air used for cooling the ultra-violet tubes and to actuate said control valve means in dependence upon the sensed temperature to maintain said equipment at a desired temperature.

With the present method and equipment it will be seen that the heat generated by the tubes and by the user is taken up by the water and is effectively "dumped" at a location remote from the atmosphere ambient to the equipment. In practice this will usually mean that where the equipment is installed in a room or solarium, the heat will be dumped outside the room or solarium.

I have discovered that by dumping the heat in this manner it is possible to control the temperature of the tubes and of a person in the space in the suntanning equipment with a high degree of accuracy and within a very small range of tolerance. Thus the temperature can be controlled to ± 0.5°C. The ability to effect such close control is of great importance when the suntanning equipment is used for therapeutic purposes for the treatment, for example, of psorias, acne and other skin conditions susceptible to treatment with ultra-violet radiation. This means that a patient can be treated for a desired length of time without having to suffer discomfort due to overheating and burning or to overcooling. This is of particular importance in the treatment of the aforesaid skin conditions because it reduces or eliminates perspiration on the part of the patient. Perspiration is deleterious not only because the salts excreted during perspiration interfere with the tanning process but also because they or the attendant humidity can aggravate the condition being treated. The perspiration can also affect the humidity in a solarium and cause discomfort for all patients.

It might be thought that the same effect as achieved by the present method could be achieved by placing the suntanning equipment in an air-conditioned atmosphere and blowing air over the tubes in the manner previously proposed. Experiments which I have conducted show that in fact this is not so, especially in solaria where the equipment is subjected to heavy and almost continuous use. In general, air conditioning does not control the temperature throughout a solarium to the same temperature all the time.  There are fluctuations in the average temperature and local fluctuations due to the presence and/or movement of people.   This means that air drawn in by blowers for use in cooling the tubes will be of fluctuating temperature and close and accurate control of the temperature of the equipment will not be possible.  Furthermore, since the air-conditioning apparatus will be relied upon to extract the heat generated by the equipment and the blowers an added burden is placed on the air-conditioning apparatus which may affect its performance elsewhere.

When a plurality of sun beds are disposed in a solarium, it is necessary for the beds to be spaced apart from one another a distance sufficient to prevent the heat generated by one sun bed affecting an adjacent sun bed.  This means that even in air-conditioned solaria there is a minimum distance which is tolerable between sun beds.  With the present method and equipment this distance can be considerably reduced as the heat generated by the equipment is extracted from the equipment and dumped at a remote location.  Thus in commercial solaria, more sun beds can be provided in a given space or the space required can be reduced, in both cases with commensurate commercial advantage.

Finally, because the temperature of the equipment is closely controlled the risk of damage due to overheating or the risk of damage from fatigue due to fluctuating temperatures is substantially reduced or eliminated which improves the life and reliability of the equipment.

In order to enable the invention to be more readily understood, reference will now be made to the accompanying drawings, which illustrate diagrammatically and by way of example some embodiments thereof, and in which:

Figure 1 is a perspective side view, partly cut away, of a sun bed,

Figure 2 is a diagrammatic representation of the sun bed shown in Figure 1, and,

Figure 3 is a view similar to Figure 2 of a modified sun bed.

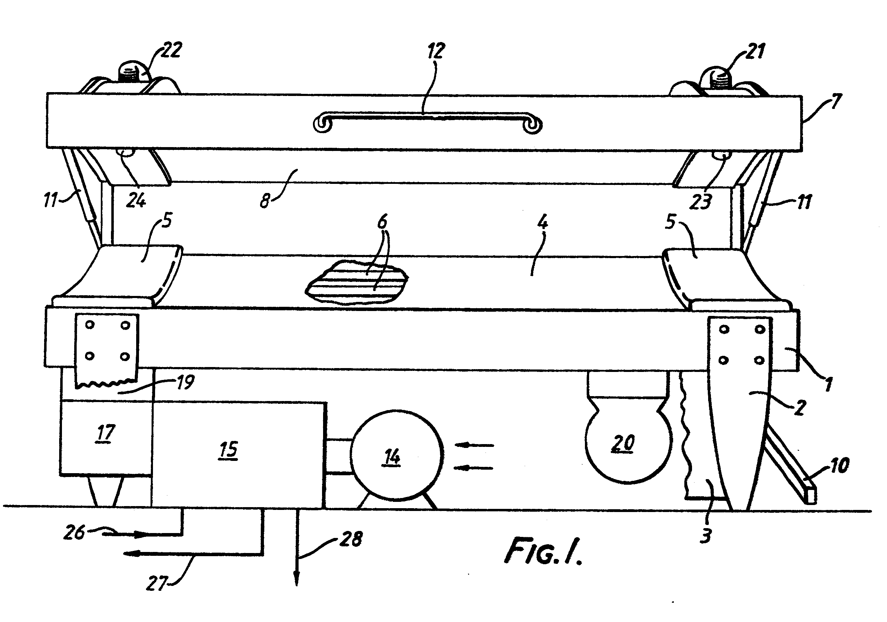

Referring now to Figures 1 and 2 of the drawings there is shown a sun bed which comprises a massive oak lower frame 1 supported on legs 2. The side of the frame between the legs 2 is covered by a decorative panel 3 which conceals the operating apparatus mounted on or beneath the frame 1. The frame 1 supports a curved sheet 4 of acrylic glass on which the user is to lie with head and feet supported by shaped cushions 5.

Beneath the sheet 4 is a plurality of ultra-violet lamps in the form of tubes 6 adapted to emit ultra violet rays, each tube being 180 cm long and the tubes being mounted in channels in a ribbed base member (not shown).

A canopy for the bed comprises an upper frame 7, also made of oak. The frame 7 mounts a curved sheet 8 of acrylic glass above which are mounted a plurality of ultra-violet lamps in the form of tubes 9 adapted to emit ultra violet rays the tubes 9 being indicated in Figure 2. The frame 7 is carried by two arms (not shown) which are pivotally mounted on substantially L-shaped supports 10 so that the upper frame 7 can be pivoted upwards and away from the lower frame 1 to permit a user to enter the space between the frames and the sheets 4 and 8 and for the upper frame 7 to be then lowered towards the lower frame 1. The upper frame is supported on the supports 10 by hydraulic struts 11, and may be moved by means of a handle 12.

In the operation of the sun bed, the user switches on the ultra-violet lamps, lays himself on the sheet 4 and cushions 5 of the lower frame, positions the upper frame a comfortable distance above himself and basks in the UV rays. However, after a period of time the tubes and the sun bed heat up and eventually the user will start to perspire. The salts excreted during perspiration interfere with the tanning process and this may lead to uneven tanning, apart of course from the discomfort felt by the user. It has been proposed to mitigate these effects by circulating the air in the room in which the sun bed is placed. However, this is merely a palliative which becomes of decreasing effect as the whole room warms up.

In accordance with the present invention means is provided for cooling air and for blowing the cooled air along the tubes 6 and 9 as well as over the body of the user so as to control the temperature of the sun bed, the user and the ambient atmosphere. To this end there are mounted below the frame 1 two blowers 14 which blow ambient air into a cooler unit 15 connected in a manner to be described to a refrigerating unit 16 (Figure 2). Air cooled in the cooler unit is passed to a plenum chamber 17 which is connected to a distributor 19 mounted in the frame 1. The distributor feeds the cold air from the cooler unit into the channels in the ribbed base in which the tubes 6 are mounted, thereby to cool the tubes as indicated in Figure 2 where the arrows show the path taken by the air. After leaving the channels the air is taken to a fan 20 from which it is supplied via flexible pipes 21 and 22 to each end of the upper frame for cooling the tubes 9, the air exiting through the sides of the frame. Air from the pipes 21 and 22 is also fed to the two outlets 23 and 24 which are arranged to blow air over the user, the air having been used to cool the tubes 6, is not as cold as when it left the cooler unit 16 and is thus more pleasant for the user.

It will be appreciated that the air leaving the outlets 23 and 24 and entering the room in which the sun bed is located is at a higher temperature than the air distributed from the plenum chamber. Therefore if the air were merely recirculated by the blowers 14 and supplied to the plenum chamber 17 the temperature in the room and of the user would eventually and fairly rapidly, depending upon the size of the room, build up to an unacceptable value at which the user would perspire and the perspiration would deleteriously affect the suntanning or therapeutic effect of the sun bed. In order to avoid such a build up of temperature, the air is cooled in the cooler unit 15. The cooler unit 15 is connected to the refrigerating unit 16, which is mounted outside the room in which the sun bed is located as indicated by the presence of a wall 25. The refrigerating unit 16 is arranged to cool a coolant, such as water, and to supply the coolant via a pipe 26 to the cooler unit 15 where it serves to cool the air supplied by the blowers 14. Coolant which has been heated by heat exchange with the air is recycled to the refrigerating unit 16 via a pipe 27. In this manner heat generated by the user and the operation of the sun bed is effectively dumped on the other side of the wall 25 and at a location remote from the sun bed. The cooler unit 15 is provided with means (not shown) for collecting water condensed as a result of cooling the air and this water can either be collected in a vessel which can be emptied periodically or, preferably, fed via a drain line 28 to the other side of the wall.

The equipment also comprises a control unit 30 which comprises on/off switches, fuses, and thermostatic controls all arranged to control the supply of and the temperature of the air fed to cool the tubes. To this end the control unit 30 contains temperature sensors 33 to measure the temperature of the cooling air leaving the tubes 6 prior to entering the fan 20. The temperature sensors are arranged to actuate, via a control line 31, a control valve 32 in the line 27 so as to control the supply of cooling fluid to the cooler unit 15. By this means it is possible to control the tmemperature of the tubes and a person using the sun bed to a required temperature witin $\pm$ 0.5°C. The temperature sensor is conveniently adjustable and provided with a scale so that the operating temperature of the sun bed can be set at a desired value. All other apparatus conventionally provided with sun tanning equipment may be housed beneath the frame 1 but, if desired, the conventional electrical chokes may be housed separately so that any heat generated in the chokes is not likely to raise the temperature of the user.

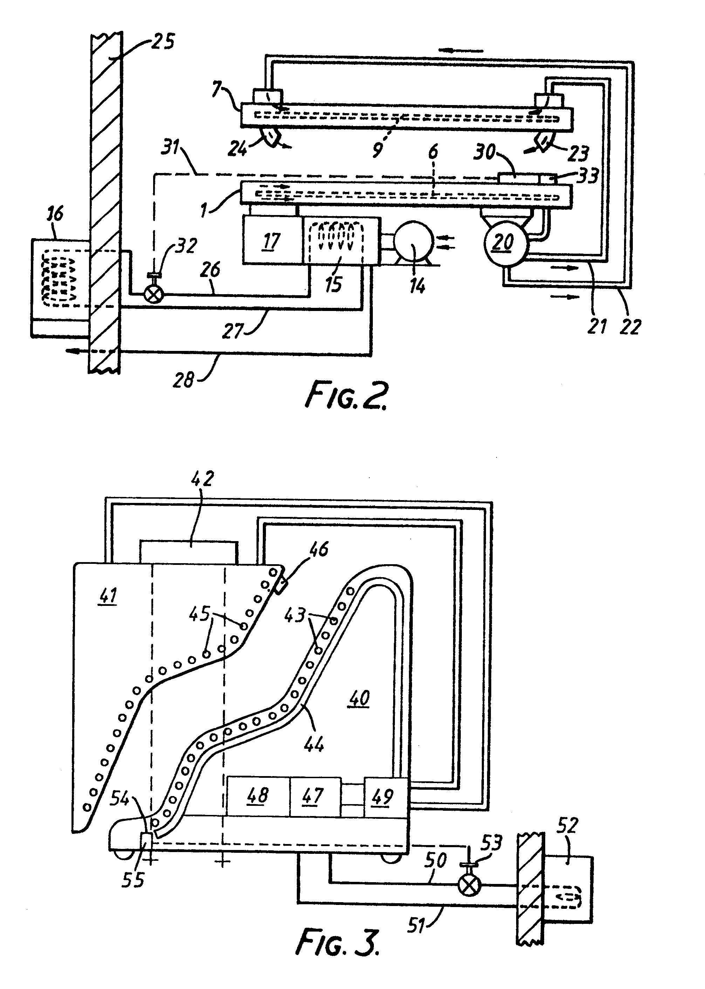

Referring now to Figure 3, there is shown a modification of the suntanning equipment just described. In this modification, the equipment is in two parts 40 and 41, the part 40 is movable on casters and the part 41 is movable up and down towards and away from the part 40 by a hydraulic mechanism indicated at 42. The part 40 is shaped to receive a user in a sitting position, as shown and the part 41 is of complementary shape. The part 40 is provided with ultra-violet lamps in the form of tubes 43 which extend transverse to the user, the tubes 43 being cooled by blowing cold air in contact with the tubes from a manifold 34 at one end of the tubes, and collecting the air in a manifold (not shown) at the other end of the tubes. The air is then passed to cool ultra-violet lamps in the form of tubes 35 in the part 31 and to blow air over the user through one or more outlets 36. In other respects the suntanning equipment shown in Figure 3 is the same as that shown in Figures 1 and 2 and has an air cooler 47 fed by blowers 48 which pass the air to a plenum chamber 49. The cooler 47 is connected via pipes 50 and 51 to a refrigerating unit 52 mounted at a remote location and the pipe 50 is valve-controlled by a valve 53 actuated by a temperature sensor 54 in a control unit 55.

The equipment shown in Figure 3 takes up less floor space than that shown in Figures 1 and 2 so that more economical use of floor space can be made in solaria and such places. Furthermore, such equipment may be particularly desirable for the treatment of elderly persons or partly handicapped persons and thus may be of particular benefit in hospitals, since it will be easier for elderly and handicapped persons to be placed in a sitting position on the part 41 rather than in a prone position.

It will be appreciated that many variations of the sun tanning equipment just described are possible and, for example, the two parts 40 and 41 shown in Figure 3 may each be mounted on casters so as to be movable towards and away from one another in which case the pipes 50 and 51 will need to be flexible hosepipes. Also the frame 7 of the equipment shown in Figures 1 and 2 may be mounted on an electric lift mechanism so that it can be moved easily towards and away from the lower frame 1. Alternatively, instead of a massive oak lower frame, the lower fram could be of light-weight construction and could be made of fibre glass, metal, e.g. tubular metal, wood or other material. Similar considerations apply to the upper frame.

Due to the close degree of control which can be exercised over the temperature of the suntanning equipment when in use, it is possible to use ultra-violet lamps of shapes and arrangements different from those which have heretofore been conventional. Thus, for example, the ultra-violet tubes may be mounted in double banks in one or both of the upper and lower frames, with the tubes of one bank located between tubes of the other bank in a staggered arrangement. By this means it is possible to enhance the treatment and to provide more compact and space-saving equipments or to adapt the equipments for particular treatments.

Furthermore, if a plurality of the equipments is provided in one area, e.g. in a solarium, it would be possible to provide one heavy duty refrigerating unit arranged to feed coolant to each equipment in the manner described, each equipment being under the individual control of its own control unit and control valve.

CLAIMS:

1.      A method of operating suntanning equipment of the kind set forth, by blowing air over the ultra-violet tubes to cool them, characterised by the steps of

1)      blowing air through heat-exchange means to cool the air,

2)      passing the cooled air in contact with the ultra-violet tubes to cool the latter,

3)      passing cooled coolant to said heat-exchange means for cooling said air by heat-exchange therewith,

4)      passing coolant warmed by such heat-exchange to cooling means,

5)      providing said cooling means at a location remote from the atmosphere ambient to the suntanning equipment,

6)      sensing the temperature of the air cooling the ultra-violet tubes, and

7)      controlling the supply of cooled coolant to said heat-exchange means in dependence upon the sensed temperature.

2.      A method as claimed in Claim 1, wherein the suply of cooled coolant to said heat exchange means is controlled so that the temperature of suntanning is controlled to a predetermined temperature $\pm$ 0.5°C.

3.    Suntanning equipment, comprising a first portion, a second portion, the portions being arranged so as to define between them a space for the reception of a user, a first plurality of ultra-violet tubes mounted in said first portion, a second plurality of ultra-violet tubes mounted in said second portion means for operating said ultra-violet tubes, whereby a user can be subjected to ultra-violet radiation when in said space,

means for blowing air over said tubes for the purpose of cooling them,

characterised by heat-exchange means to cool the air suplied by said air-blowing means, cooling means for providing a supply of cooled coolant, said cooling means being located remote from the atmosphere ambient to said equipment, first conduit means for feeding cooled coolant to said heat-exchange means, second conduit means for recycling coolant from said heat-exchange means back to said cooling means, control valve means in said first conduit means for regulating the supply of cooled coolant to said heat-exchange means, and temperature sensing means to sense the temperature of the air used for cooling the ultra-violet tubes and to actuate said control valve means in dependence upon the sensed temperature to maintain said equipment at a desired temperature.

4.      Equipment as claimed in Claim 3, wherein the first portion comprises a base shaped to receive a user in a prone position and the second portion comprises a cover mounted so as to be movable towards and away from the first position.

5.      Equipment as claimed in Claim 3, wherein the first portion comprises a base shaped to receive a user in a sitting position and the second portion is shaped complementary thereto.

6.      Equipment as claimed in Claim 4 or 5, wherein fan means is provided for supplying air, used to cool the ultra-violet tubes of said first portion, to cool the ultra-violet tubes of said second portion.

7.      Equipment as claimed in Claim 4, 5 or 6, wherein the temperature sensing means is located to sense the temperature of the air which has been used to cool said first portion.

8.      Equipment as claimed in any one of Claims 3 to 7, wherein means is provided for collecting or removing water condensed from the air in said heat-exchange means.

9.      A solarium comprising a plurality of the suntanning equipments claimed in any one of Claims 3 to 8.

10.     A solarium as claimed in Claim 9, wherein one common cooling means is provided for the plurality of suntanning equipments.

FIG.I.

0208392

FIG. 2.

FIG. 3.

| | DOCUMENTS CONSIDERED TO BE RELEVANT | | EP 86302447.7 |
|---|---|---|---|
| Category | Citation of document with indication, where appropriate, of relevant passages | Relevant to claim | CLASSIFICATION OF THE APPLICATION (Int. Cl.4) |
| Y | <u>DE - A1 - 3 231 270</u> (WOLFF) | 1,3 | A 61 N 5/06 |
| A | * Abstract; page 1; claim 1; fig. 3 * | 4 | F 21 V 29/00 |
| | -- | | |
| Y | <u>GB - A - 1 380 020</u> (COMISSARIAT) | 1,3 | |
| A | * Page 1, lines 70-81; fig. 2 * | 2,5-7 | |
| | -- | | |
| X | <u>EP - A1 - 0 026 239</u> (KÖPPEN) | 1,3 | |
| A | * Abstract; page 7, lines 25-28; page 2, claim 10 * | 4-6 | |
| | -- | | |
| Y | <u>DE - A1 - 3 303 795</u> (JK-JOSEF KRATZ) | 1,3 | |
| A | * Abstract; page 1, claim 1; fig. 1 * | 4,9,10 | |
| | -- | | |
| Y | <u>US - A - 3 947 679</u> (FRENGER) | 1,3 | TECHNICAL FIELDS SEARCHED (Int. Cl.4) |
| | * Abstract; column 6, lines 8-16; fig. 1 * | | A 61 N |
| | -- | | F 21 V |
| A | <u>DE - A1 - 2 622 993</u> (SUN CHEMICAL CORP.) | 1,3 | |
| | * Fig. 1 * | | |
| | -- | | |
| Y | <u>DE - A1 - 3 303 794</u> (JK-JOSEF KRATZ) | 1,3 | |
| A | * Abstract; fig. 1 * | 4,9,10 | |
| | -- | | |

The present search report has been drawn up for all claims

| Place of search | Date of completion of the search | Examiner |
|---|---|---|
| VIENNA | 08-08-1986 | NEGWER |

**European Patent Office**

**EUROPEAN SEARCH REPORT**

. Application number

| DOCUMENTS CONSIDERED TO BE RELEVANT | | | EP 86302447.7 |
|---|---|---|---|
| Category | Citation of document with indication, where appropriate, of relevant passages | Relevant to claim | CLASSIFICATION OF THE APPLICATION (Int. Cl.4) |
| Y | US - A - 3 869 605 (DAVIS) <br> * Abstract; column 3, lines 12-18; fig. 1,2 * <br> -- | 1,3 | |
| A | DE - A1 - 3 035 624 (FREI) <br> * Fig. 6-9 * <br> -- | 1,3,5, 9,10 | |
| A | DE - A1 - 2 804 228 (WOLFF) <br> * Fig. 9,12 * <br> ---- | 1,4,5, 9,10 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 08-08-1986 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82